# FASCICULE DE BREVET EUROPEEN

(11) **EP 0 651 649 B1**
(45) Date de publication et mention de la délivrance du brevet: **17.12.1997**
(21) Numéro de dépôt: 93916011.5
(22) Date de dépôt: 19.07.1993
(51) Int. Cl.: A61K 38/00, A61K 9/00

(54) **PROCEDE D'ADMINISTRATION DE SOLUTONS DE FACTEUR DE STIMULATION DES COLONIES GRANULOCYTAIRES**
VERFAHREN ZUR VERABREICHUNG EINER LÖSUNG VON GRANULOZYTEN-KOLONIE-STIMULIERENDEM FAKTOR
GRANULOCYTE COLONY-STIMULATING FACTOR SOLUTION DELIVERY METHOD

(30) Priorité: 23.07.1992 FR 9209079
(43) Date de publication de la demande: 10.05.1995
(73) Titulaire: RHONE-POULENC RORER S.A., 92160 Antony (FR)
(72) Inventeur: BOUSSEAU, Anne, F-75011 Paris (FR); FRYDMAN, Armand, F-91370 Verrières-le-Buisson (FR); PLARD, Jean-Paul, F-94400 Vitry (FR); SPENLEHAUER, Gilles, F-94230 Cachan (FR); VEILLARD, Michel, F-92330 Sceaux (FR)
(74) Mandataire: Savina, Jacques
(86) Numéro de dépôt international: FR9300731
(87) Numéro de publication internationale: WO9402164

(56) Documents cités:
- EP-A- 0 104 933
- WO-A-90/00060
- DE-A- 3 723 781
- PROCEEDINGS OF THE NATIONAL ACADEMY OF SCIENCES OF USA. vol. 84 , Avril 1987 , WASHINGTON US pages 2484 - 2488 A.M. COHEN ET AL. 'IN VIVO STIMULATION OF GRANULOPOIESIS BY RECOMBINANT HUMAN GRANULOCYTE COLONY-STIMULATING FACTOR.'

## Description

La présente invention concerne un nouveau procédé d'administration de solutions contenant un facteur de stimulation des colonies de granulocytes. En particulier cette invention concerne un procédé d'administration de solutions aqueuses de G-CSF.

La chimiothérapie utilisée fréquemment dans le traitement des maladies cancéreuses provoque la destruction des cellules cancéreuses mais présente aussi souvent des effets secondaires cliniques liés à ce traitement. Ainsi au cours de ces traitements est apparue l'existence de maladies infectieuses dues à des microorganismes résistant à toute thérapie antibiotique. En plus au cours de ces traitements chimiothérapeutiques une partie des défenses naturelles des organismes vivants sont détruites ce qui explique l'apparition des maladies infectieuses chez ces organismes.

Afin d'éviter au maximum l'apparition des ces maladies infectieuses liées à la chimiothérapie il a été proposé d'utiliser une substance qui active les capacités de défense naturelle de l'organisme. Dans chaque organisme il existe des systèmes de défense qui chez l'homme sont constitués en majorité de leucocytes. Ces leucocytes assurent la phagocytose des agents infectieux ou participent à leur destruction et par là évitent la multiplication des microorganismes nuisibles à l'intérieur de l'organisme malade. Parmi les substances qui favorisent la prolifération ou la multiplication des leucocytes est apparu récemment le facteur de stimulation des colonies de granulocytes. Ce facteur a été protégé dans la demande de brevet européen publiée par exemple sous le numéro EP 237 545.

Ce facteur de stimulation des colonies de granulocytes est administré à 'homme ou à l'animal à des doses très faibles de l'ordre de 0,1 à 500 µg pour un homme adulte. L'administration de doses aussi faibles pose un problème de dilution du principe actif qu'il a falllu surmonter. En effet ce principe actif qui est un polypeptide présente l'inconvénient de s'adsorber sur les parois des récipients avec lesquels il a été mis en contact que ce soit des seringues ou des flacons de perfusion. Le problème lié à cette adsorption a été résolu dans la demande de brevet britannique publiée sous le numéro GB 2 193 631.

Il est connu par exemple selon cette demande de brevet anglais que l'on peut stabiliser la solution du facteur de stimulation des colonies de granulocytes par adjonction à la solution aqueuse d'un protéine concurrente quant à l'adsorption sur les parois du récipient; protéine constituée par exemple: d'albumine, d'un polysaccharide couplé à un agent surfactant non ionique.

Ces solutions lorsqu'elles sont administrées chez l'animal, par voie sous cutanée, provoquent deux jours seulement après l'injection, une augmentation de cinq fois la quantité de polynucléaires neutrophiles, mais cette augmentation se réduit à environ deux fois la normale, huit jours après l'injection, tout ceci étant évalué par rapport à des témoins n'ayant reçu aucun traitement.

Une méthode d'administration à l'aide de particules présentant une libération prolongée a été proposée dans la demande de brevet EP 263 490. Cette méthode permet grace à l'administration de particules polymériques à base de polymère d'acide polylactique ou de copolymère d'acide lactique et d'acide glycolique contenant le facteur de stimulation des colonies de granulocytes, d'éviter l'administration quotidienne de solutions aqueuses de ce dit facteur. Ces particules permettent d'obtenir un effet pratiquement équivalent à celui obtenu avec l'injection sous cutanée quotidienne de solutions aqueuses du dit facteur. L'effet maximal est ainsi maintenu sur toute la période de libération des microparticules qui peut atteindre deux semaines.

Dans la demande internationale WO 90/000060 a été décrite une matrice de collagène utile pour la distribution prolongée d'agents bioactifs, notamment le G-CSF. Cet implant est destiné à être placé sur une plaie.

Dans Proc. Natl. Acad. Sci., 84, 2484 (1987) a été décrite une étude de l'activité in vivo du rhG-CSF par voie sous-cutanée en administration journalière unique ou par voie intra-veineuse en continu.

Il est apparu de façon tout à faite étonnante que lorsque la solution aqueuse de facteur de stimulation était introduite par voie sous cutanée, en continu, chez l'animal ou chez l'homme, l'effet multiplicateur de la teneur en neutrophiles était accru de façon considérable. Par contre il est apparu que la même dose injectée par voie cutanée en une injection unique quotidienne entrainait une augmentation six fois moindre celle provoquée par l'administration de la même dose journalière en continu. Cet effet tout à fait surprenant présente une conséquence tout à fait favorable pour le traitement à l'aide de ce genre de principes actifs, elle permet ainsi pour le même effet thérapeutique, c'est à dire pour la même augmentation du taux sanguin de neutrophiles de diminuer les doses injectées par au moins un facteur de dix et encore plus avantageusement par un facteur de vingt.

La présente invention consiste donc à injecter les solutions décrites dans le brevet britannique publié sous le numéro GB 2 163 631. Ces solutions présentent de préférence la composition suivante :

| | |
|---|---|
| - facteur de stimulation des colonies de granulocytes | 250 µg |
| - sérumalbumine | 1 mg |
| - tensioactif non ionique | 0,1 mg |
| - saccharide | 50 mg |
| - phosphate disodique | 0,8 mg |
| - phosphate monosodique | 3,6 mg |
| - chlorure de sodium | 3,1 mg |
| - eau | 660 mg |

Le tensioactif utilisé est de préférence le polysorbate 80. Le saccharide utilisé est de préférence le mannitol. Il est bien évident que la composition n'est pas strictement figée à la répartition quantitative évoquée mais qu'elle peut varier en concentration dans de larges limites, le volume injecté sera inversement proportionnel à la concentration de la solution.

Pour une réponse optimale il est conseillé d'administrer une dose comprise entre 0,05 et 2 µg / kg / h. Cette administration peut être réalisée d'une manière pratique à l'aide d'une pompe implantée dans le tissu sous cutané ou à l'aide d'implant permettant une libération continue et régulière du principe actif.

La présente invention est plus complètement décrite à l'aide des exemples suivants.

### EXEMPLE

### Conditions expérimentales

### 1) Matériel

On utilise des minipompes osmotiques ALZET ayant une capacité de 200 µl, remplies avec une quantité suffisante de principe actif pour permettre une libération régulière et constante pendant 8 jours, à raison de 1 µl/heure de solution, soit 10µg/24H/animal, du facteur de stimulation.

### 2) Animaux

On utilise 40 rats (20 mâles et 20 femelles) agés de 7 à 8 semaines pesant de 180 à 200g
principe actif : lyophilisat de 250 µg de facteur stimulant dissous dans 0,66ml d'eau stérile pour injections
matériel : pompes osmotiques ALZET remplies avec une quantité suffisante de principe actif pour permettre une libération du facteur de stimulation, pendant huit jours à raison de 10 µg/24 H par animal.

Les quarante animal impliqués dans l'essai sont ainsi répartis:
A - 10 sont des témoins qui reçoivent uniquement une injection sous cutanée quotidienne (8 jours) d'une solution de chlorure de sodium.
B - 10 sont des témoins de technique opératoire, sur lesquels, on pratique une incision dorsoscapulaire, sans introduction de minipompe, avec suture.
C - 10 autres reçpovent une injection sous cutanée quotidienne, de 24µl/jour, de solution de facteur de stimulation, pendant 8 jours.
D - les dix derniers reçoivent une implantation sous cutanée, en région dorsoscapulaire, d'une minipompe ALZET, qui délivre 1µl/H de solution de facteur de stimulation, pendant 8 jours.

Les résultats de la neutropoïèse sont indiqués sur les tableaux 1 et 2.

**tableau 1**

| NEUTROPOIESE | | | | | |
|---|---|---|---|---|---|
| MALES | Nombre de Neutrophiles/mm³ | | | | |
| | T0 | 2J | 3J | 5J | 8J |
| A | 800 (350)* | 1900 (1300) | 3700 (3000) | 5500 (2300) | 3500 (1100) |
| B | 1600 (600) | 2450 (700) | 2400 (1200) | 4200 (2400) | 3560 (1300) |
| C | 1200 (650) | 8500 (4500) | 7050 (2500) | 3300 (3800) | 7500 (1700) |
| D | 1800 (800) | 17000 (3400) | 26000 (5600) | 26600 (7800) | 48500 (20000) |

| | | | | | |
|---|---|---|---|---|---|
| * représente l'écart-type | | | | | |

**tableau 2**

| NEUTROPOIESE | | | | | |
|---|---|---|---|---|---|
| FEMELLES | Nombre de Neutrophiles/mm³ | | | | |
| | T0 | 2J | 3J | 5J | 8J |
| A | 460 (400) | 1900 (800) | 1600 (600) | 1800 (600) | 1500 (700) |
| B | 890 (400) | 1100 (600) | 2500 (1400) | 1900 (800) | 2350 (1200) |
| C | 500 (300) | 6600 (3000) | 7400 (1900) | 4100 (2000) | 5000 (1700) |
| D | 1100 (400) | 18000 (3000) | 31000 (10000) | 26000 (7200) | 46000 (10000) |

Le même essai qu'à l'exemple précédent a été réalisé avec des solutions moins concentrées de facteur de stimulation, des concentrations de 0,3 µg/24 H (lot D1), 1 µg/24 H (lot D2) et 3 µg/24H (lot D3) ont été essayées sur des animaux similaires et dans les mêmes conditions qu'à l'exemple précédent. Le résultats de la neutropoièse sont indiqués sur le tableau 3.

**tableau 3**

| NEUTROPOIESE | | | | |
|---|---|---|---|---|
| Groupes | Nombre de Neutrophiles/mm³ | | | |
| | T0 | 3J | 5J | 8J |
| A | 1800 (400) | 2000 (800) | 2000 (700) | 2200 (500) |
| B | 2700 (2400) | 2700 (700) | 3200 (1000) | 2700 (1300) |
| D1 | 1500 (1300) | 5800 (800) | 4900 (1600) | 5400 (1800) |
| D2 | 1200 (700) | 10300 (4500) | 10100 (5100) | 18700 (4400) |
| D3 | 1600 (500) | 11000 (4000) | 8500 (4000) | 30000 (7100) |

## Revendications

1. Utilisation de facteur stimulant les colonies granulocytaires, pour la préparation d'un médicament destiné au traitement des risques de maladies infectieuses au cours des traitements chimiothérapeutiques, caractérisée en ce que ledit médicament est administré en continu, par voie sous cutanée.

## Claims

1. Use of granulocyte-colony stimulating factor for the preparation of a medicament for use in the treatment of risks of infectious diseases during chemotherapeutic treatments, characterized in that the said medicament is delivered continuously subcutaneously.

## Patentansprüche

1. Verwendung von Granulocyten-Kolonien stimulierendem Faktor für die Herstellung eines Arzneimittels zur Behandlung von Risiken infektiöser Erkrankungen im Verlauf von chemotherapeutischen Behandlungen, dadurch gekennzeichnet, daß das genannte Arzneimittel kontinuierlich auf subkutanem Wege verabreicht wird.
